# EUROPÄISCHE PATENTSCHRIFT

(11) **EP 1 224 904 B1**
(45) Veröffentlichungstag und Bekanntmachung des Hinweises auf die Patenterteilung: **08.03.2006**
(21) Anmeldenummer: 01130320.3
(22) Anmeldetag: 19.12.2001
(51) Int. Cl.: A61B 5/00, A61M 16/04

(54) **Endoskop für die Notfallintubation**
Endoscope for emergency intubation
endoscope pour l'intubation en urgence

(30) Priorität: 09.01.2001 DE 10100533
(43) Veröffentlichungstag der Anmeldung: 24.07.2002
(73) Patentinhaber: Karl Storz GmbH & Co. KG, 78532 Tuttlingen (DE)
(72) Erfinder: Pilvisto, Tonis, 13912 Tallinn (EE)
(74) Vertreter: Eder, Christian

(56) Entgegenhaltungen:
- US-A- 3 557 780
- US-A- 5 441 483
- US-A- 5 531 686
- US-A- 5 549 542
- US-A- 5 842 973

## Beschreibung

Die Erfindung betrifft ein Endoskop gemäß dem Gattungsbegriff des Anspruchs 1.

Solche Vorrichtungen finden Anwendung auf einer Vielzahl von Gebieten, wie in der Medizin, beispielsweise in der Endoskopie, insbesondere für die Notfallintubation, in der Technik, beispielsweise endoskopartige Werkzeuge mit endoskopartigem flexiblen und formbaren Schaft und allgemeiner Anwendung, wie der kontrollierten Bewegung von Gliedmaßen, insbesondere Gliedmaßen eines Menschen.

Unter Intubation wird das Einführen eines aus Gummi oder Kunststoff bestehenden Schlauchs (Tubus bzw. Katheder) in den Kehlkopf und weiter in die Trachea des Individuums verstanden. Sie dient der Aufrechterhaltung eines effektiven, u.a. für die Sauerstoffversorgung der Organe lebensnotwendigen Gasaustauschs, der normalerweise durch Atmung erfolgt. In Situationen, in denen der Patient infolge Krankheit, Verletzung oder Medikamentengabe, z.B. bei der Durchführung einer Narkose, nicht mehr in der Lage ist, die erforderliche Atemarbeit zu bewältigen, muss eine Beatmung durchgeführt werden. Voraussetzung für diese Beatmung ist eine sichere Verbindung zwischen Beatmungsgerät und den Atemwegen des Patienten. Um eine ausschließliche Belüftung der Atmungsorgane zu erreichen, ohne dass Luft über die Speiseröhre in den Verdauungstrakt gelangt, wird der Tubus mit seinem distalen Ende über den Mund oder die Nase in die Luftröhre geschoben und dort so platziert, dass beide Lungenflügel belüftet werden. Am proximalen Ende wird der Tubus über ein genormtes Ansatzstück (Konnektor) mit dem Schlauchsystem des Beatmungsgeräts verbunden.

Liegt die Tubusspitze nicht in der Trachea, werden die Lungenflügel nicht belüftet. Das durch die Lunge strömende Blut wird dann nicht ausreichend mit Sauerstoff angereichert, und die Versorgung der Organe mit Sauerstoff bricht zusammen. Je nach Dauer der ungenügenden Versorgung mit Sauerstoff sind neben einer vollständigen Wiederherstellung sämtlicher Körperfunktionen bleibende Hirnschäden (z.B. Koma) oder der Tod infolge Herzstillstand möglich. Dieselben Folgen ergeben sich bei Fehlintubationen, worunter Intubationsmanöver verstanden werden, bei denen der Tubus nicht richtig platziert wird und z.B. die Tubusspitze in der Speiseröhre anstatt in der Luftröhre liegt.

Zur sicheren Platzierung der Tubusspitze gibt es mehrere Methoden und Tubusformen. Bei Patienten ohne anatomische oder pathologische Besonderheiten ist die Intubation in der Regel mit den gebräuchlichen Methoden, hauptsächlich der laryngoskopischen Intubation, einfach und schnell durchführbar. Schwierigkeiten ergeben sich allerdings z.B. bei Vorliegen von pathologischen Veränderungen oder anatomischen Besonderheiten, wodurch dann am nicht mehr atmenden Patienten die Kombination der laryngoskopischen Intubation mit der fiberoptischen Intubation oder die Anwendung von diesen speziell zugeordneten Geräten erfolgt.

In der Notfallintubation, also in der Regel der Intubation eines bewußtlos gewordenen Menschen, der Gefahr läuft, dass sich seine Lunge mit Wasser füllt, muss zur Verringerung des Verletzungsrisikos der Lunge möglichst schnell Luft von außen zugeführt werden.

Dies geschieht bei einem Notfall, d.h. am Unfallort und in der Regel nicht in einem Krankenhaus, durch eine laryngoskopische Intubation, wobei mittels eines Laryngoskops unter Anhebung des Zungengrundes und des Kehlkopfdeckels der Rachen des Patienten geöffnet wird, um freie Sicht auf den Kehlkopfeingang, die Stimmritze zu erhalten. Ist dabei die Stimmritze nur teilweise einsehbar, dann ist die Einführung des Tubus schwierig. Man behilft sich damit, dass die Form des Tubus in seiner Längsachse so lange verändert wird, bis die Tubusspitze den Tracheaeingang sicher erreichen kann. Dies geschieht bisher durch eine innere Schienung des Tubus mittels eines in diesen eingesetzten Führungsstabs in Form eines mit Kunststoff ummantelten biegsamen Drahts, der nach dem Biegevorgang so stabil ist, dass er seine Form in der Längsachse auf den elastischen Tubus überträgt. Er ist im Tubus in der Längsachse verschiebbar, so dass er auch mit seiner weichen Spitze aus dem distalen Tubusende herausragen kann. Je nach den anatomischen Gegebenheiten wird der Führungsstab so gebogen, dass seine Spitze durch die Stimmritze geschoben und anschließend die Tubusspitze durch Schieben über den Führungsstab in der Trachea platziert werden kann. Ist aufgrund anatomischer Schwierigkeiten die Stimmritze nicht einsehbar, so dass der Weg des Tubus bzw. Führungsstabs beim Vorschieben nicht beobachtet werden kann, liegt bei erhöhtem Verletzungsrisiko eine stark erniedrigte Trefferquote vor. Für diese Fälle gibt es zwar Spezialinstrumente, die auch unter schwer einstellbaren Bedingungen einen Blick auf die Stimmritze ermöglichen sollen, doch wird bei deren Anwendung wegen der meist beengten Verhältnisse die Sicht durch den Tubus häufig behindert. Die Anwendung der laryngoskopischen Intubation ist daher in schwierigen Fällen nicht optimal.

In derartigen Fällen wird daher vorzugsweise die fiberoptische Intubation angewendet, bei der man sich zum Auffinden des Tracheaeingangs eines Endospkops der eingangs bezeichneten Gattung bedient, um das Beobachtungsfeld auszuleuchten und einzusehen. Außerdem kann mittels einer am Halteteil des Endoskops montierten Mechanik die Lage der Endoskopspitze verändert und dadurch unter Sicht durch die Stimmritze geschoben werden. Der vorher auf den Endoskopschlauch aufgeschobene Tubus wird dann bis in die Trachea vorgeschoben, worauf das Endoskop aus dem richtig platzierten Tubus herausgezogen und letzterer am Kopf des Patienten fixiert und an das Beatmungsgerät angeschlossen wird.

Auch die fiberoptische Methode ist nicht in allen Fällen optimal durchführbar. Sie ist insbesondere beim liegenden Patienten mit erschlaffter Muskulatur sehr schwierig, weil der Zungengrund nach hinten fällt, wodurch der Weg zur Trachea verlegt ist. Da außerdem bei der fiberoptischen Intubation eine Hand zum Führen des Endoskops, meistens mittels des umgebenden Tubus, und eine zweite Hand zum Bedienen der Endoskopmechanik benötigt wird, ist für das Anheben des Zungengrunds mittels eines Laryngoskops eine Hilfsperson erforderlich, die bei einem Notfall nicht unbedingt vorhanden ist.

In der EP 0742026 A wurde deshalb ein flexibles und gleichzeitig formbares Endoskop mit Betrachtungsoptik vorgeschlagen, über das ein Tubus geschoben werden kann. Die Formbarkeit und die Flexibilität sollte hierbei durch einen Gliederstab, dessen einzelne aneinandergrenzende Glieder mit konvexen oder konkaven Oberflächen durch Zug- oder Druckkraft verspannbar sind, erfolgen. Nachteiligerweise stellte sich in Herstellungsversuchen heraus, dass dieses Prinzip infolge der schwer kontrollierbaren Reibungskräfte sehr aufwendig ist und eine ausreichende Flexibilität bei gleichzeitiger einstellbarer Starrheit hiermit nicht realisierbar ist.

Aus der US 5842973 ist ein nasales Intubationsgerät bekannt, welches einen L-förmigen starren Stahlschaft mit einer distalen steuerbaren Spitze aufweist.

Aus der US 5531686 ist weiterhin ein Lenkmechanismus für Katheder bekannt, bei dem das distale Ende eines Lenkschaftes ausgelenkt werden kann.

Der Erfindung liegt deshalb die Aufgabe zugrunde, kostengünstig und auf einfache Weise ein Endoskop zu schaffen, das die vorstehend geschilderten Nachteile überwindet, und zumindest in Teilbereichen einen formbaren und starren Schaft aufweist, der nach dem Lösen in zumindest diesen Teilbereichen in einen flexiblen Zustand übergeht.

Diese Aufgabe wird erfindungsgemäß mit den Merkmalen des Anspruchs 1 gelöst. Durch Anordnung von länglichen biegbaren Zug- und/oder Schubmittel bzw. deren distalen Enden am Schaft in unterschiedlichen Abständen vom proximalen Ende, kann der Schaft im flexiblen, also nicht festgestellten Zustand von Hand in eine gewünschte Form gebracht werden, die durch ein Feststellen der Zug- und/oder Schubmittel starr wird. Hierbei greifen die Zug- und/oder Schubmittel zumindest in Schub- oder Zugrichtung beschränkt an dem Schaft an, wobei durch eine beidseitige Begrenzung die Stabilität der Starrheit erhöht werden kann. Durch die steuerbare Endoskopspitze ist hierbei trotz Fixierung einer vorgeformten Form des Schaftes eine Einführen, insbesondere in Extremsituationen oder für weniger erfahrende Ärzte erleichtert, da während eines Einführens, beispielsweise bei einer Notfallintubation in die Luftröhre, über die Optik der jeweils weitere Weg beobachtet werden kann und die Spitze an jeweilige Windungen angepasst werden kann. Hierdurch ist es beispielsweise selbst bei einem Riss der Luftröhre möglich, an der Reißstelle den entstehenden Versatz der Luftröhrenenden (beispielsweise mehrere Millimeter) durch eine entsprechende Bewegung der Spitze auszugleichen und ein einfaches und schnelles Einführen eines Tubus bzw. Luftröhrenkatheters (über den Schaft geschoben) auch über eine solche Extremstelle zu gewährleisten.

In einer vorteilhaften Ausführungsform der Erfindung ist die Spitze der Vorrichtung nur in einer Ebene auf- und ab- bzw. nach links und nach rechts steuerbar, wobei sich diese Bewegung vorteilhafterweise bereits durch eine geringe Anzahl von Steuermitteln erreichen lässt, wobei der Querschnitt des Schaftes innerhalb dessen die Steuermittel untergebracht sind entsprechend klein gehalten werden kann.

Durch Drehung der gesamten Vorrichtung um ihre Längsachse können jedoch beliebige Richtungen bzw. Kopfpositionen erreicht werden, wobei eine solche Drehung selbst im Einsatz der Notfallintubation in einem gewissen Bereich ohne Verletzung der Luftröhre möglich ist. Selbstverständlich ist es auch denkbar bei entsprechenden Abmessungen weitere Steuermittel im Schaft bis zur Spitze unterzubringen, so dass eine Steuerbarkeit der Vorrichtungsspitze in allen Richtungen, wie bei herkömmlichen flexiblen Endoskopen, ermöglicht wird. Da in der bevorzugten Ausgestaltung der Erfindung die Bewegbarkeit bzw. Formbarkeit des Schaftes ebenfalls auf eine Bewegung (beispielsweise S-förmig) innerhalb derselben Ebene, welche die Längsachse der Vorrichtung einschließt, wie die Steuerbarkeit der Spitze eingeschränkt sein kann, ist die Starrheit in anderen Ebenen und damit die Unempfindlichkeit gegen Stauchungen oder Verwindungen, insbesondere bei einer Drehung um die Längsachse der Vorrichtung besonders hoch. Die gesamte Vorrichtung stellt dann gegen Bewegungen in andere Richtungen einen von Haus aus starren bzw. festen Körper dar.

In einer Ausführungsform nach der Erfindung können die Zug- und/oder Schubmittel, beispielsweise Stahllitzen, in Führungselementen in Längsrichtung am Schaft beweglich gelagert sein. Hierdurch erhöht sich vorteilhafterweise die Stabilität der Starrheit, da die Weglänge Zug- und/oder Schubmittel durch die Ortsfestigkeit dieser Lagerung exakt bestimmt ist.

In weiterer Ausgestaltung der Erfindung sind die Zug- und/oder Schubmittel innerhalb des Schaftes (1) im Querschnitt ringförmig an dessen Innenumfang gelagert, so dass sich vorteilhafterweise deren Hebelwirkung und damit die Stabilität der Starrheit, die sich mit zunehmendem Abstand von der Mittelachse vergrößert, verbessert.

In weiterer Ausgestaltung der Erfindung sind innerhalb des Schaftes im Querschnitt gegenüberliegende, mit dem Schaft wenigstens in Teilbereichen fest verbundene seitliche Führungen angeordnet. Hierdurch wird vorteilhafterweise eine unerwünschte Biegung des Schaftes innerhalb der durch seitliche Führungen bestimmten Ebene verhindert.

In weiterer Ausgestaltung der Erfindung ist der Kanal als Kanal für einen Lichtleiter und einen Bildleiter und/oder als ein Instrumentenkanal ausgebildet ist. Hierdurch kann vorteilhafterweise während einer Einführung der endoskopartigen Vorrichtung in einen nicht einsehbaren Bereich das Erreichen eines Zielpunktes überprüft und erleichtert werden.

In weiterer Ausgestaltung der Erfindung greifen Zug- und/oder Schubmittel am Schaft in axialer Richtung paarweise in im Wesentlichen gleichen Abständen vom proximalen Ende an. Hierdurch kann durch ein Zusammenwirken der Schub- und Zugkräfte eines Paares die Stabilität erhöht werden. Hierbei können Zug- und/oder Schubmittelpaare am Schaft punktsymmetrisch angeordnet sein, um bei einem frei beweglichen Schaft ohne seitliche Begrenzung durch ihren maximalen Abstand voneinander vorteilhafterweise die optimale Beharrungskraft auzuüben.

Bei einer seitlichen Begrenzung der Bewegung kann es aber auch für eine optimale Kraftwirkung von Vorteil sein, Zug- und/oder Schubmittelpaare am Schaft symmetrisch zur horizontalen oder vertikalen Achse anzuordnen, also jeweils symmetrisch zu der durch die seitlichen Führungen bestimmten Ebene.

Weitere vorteilhafte Ausführungsformen der Erfindung ergeben sich aus den abhängigen Ansprüchen.

Die Erfindung wird nachfolgend anhand eines in der Zeichnung dargestellten Ausführungsbeispiels näher erläutert. In der Zeichnung zeigen:
- Fig. 1: einen Längsschnitt durch einen Schaft eines Endoskops gemäß der Erfindung;
- Fig. 2: einen Querschnitt durch den Schaft nach Fig. 1;
- Fig. 3: eine perspektivische schematische Darstellung des Aufbaus eines Teilbereiches des Schafts nach Fig. 1;
- Fig. 4: eine Seitenansicht in teilweise aufgebrochener Darstellung des Endoskops gemäß der Erfindung und
- Fig. 5: eine Vorderansicht auf die Einzelheit nach Fig. 4.
- Fig. 6: eine Seitenansicht in teilweise aufgebrochener Darstellung einer weiteren Ausführungsform eines Endoskops gemäß der Erfindung und
- Fig. 7: eine Vorderansicht auf die Einzelheit nach Fig. 6.

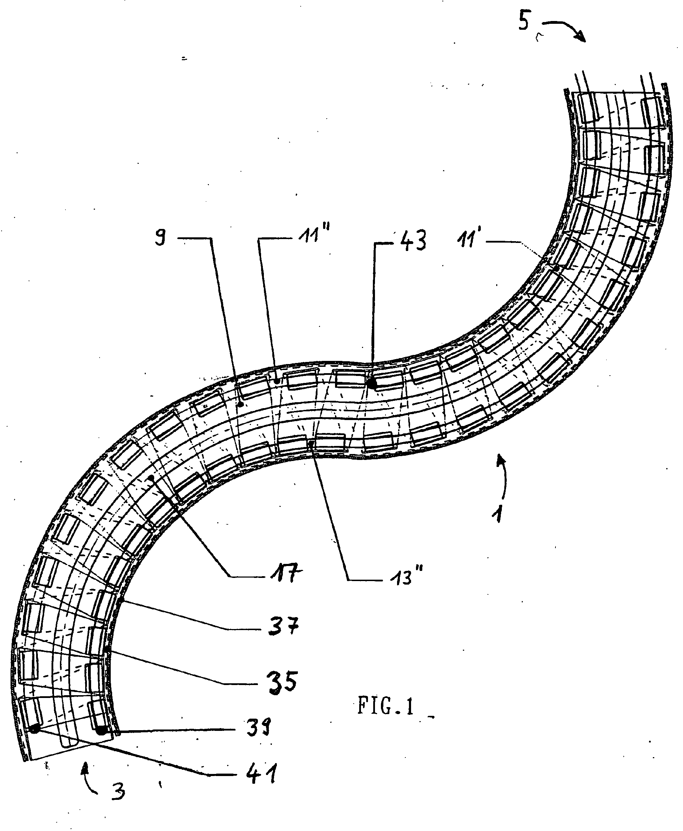

In Fig. 1 bis Fig. 5 ist eine erste Ausführungsform nach der Erfindung dargestellt, die der endoskopartigen Vorrichtung der unveröffentlichten deutschen Patentanmeldung 199 32 022.5 entspricht.

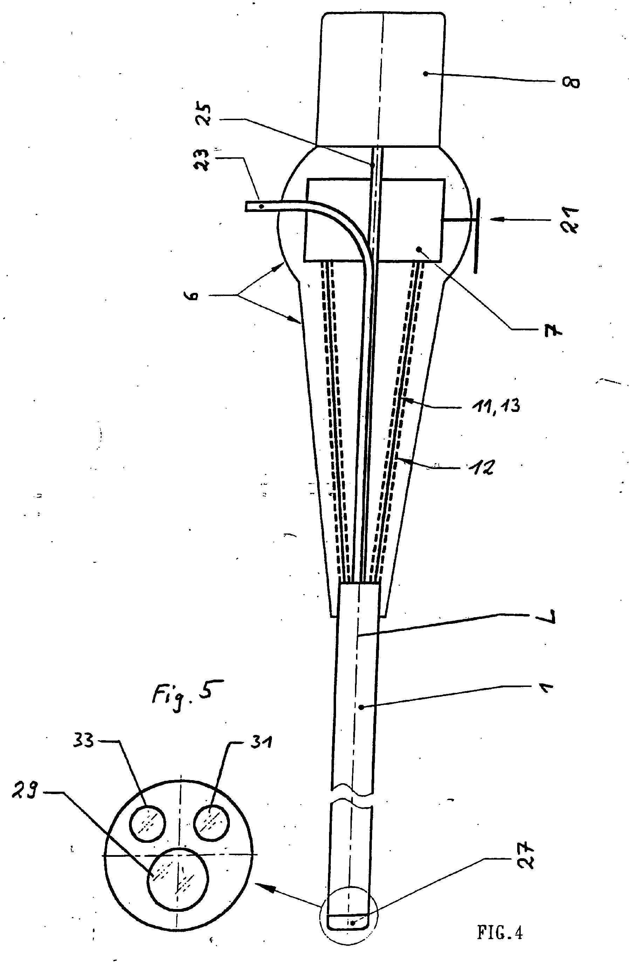

Fig. 1 zeigt einen Schaft 1 eines erfindungsgemäßen Endoskops mit einem distalen Ende 3 und einem proximalen Ende 5. An das proximale Ende 5 des Schaftes 1 schließt sich ein in Fig. 4 dargestelltes Gehäuse 6 mit Halteteil 7 (siehe Fig. 4) und einem Okular 8 an.

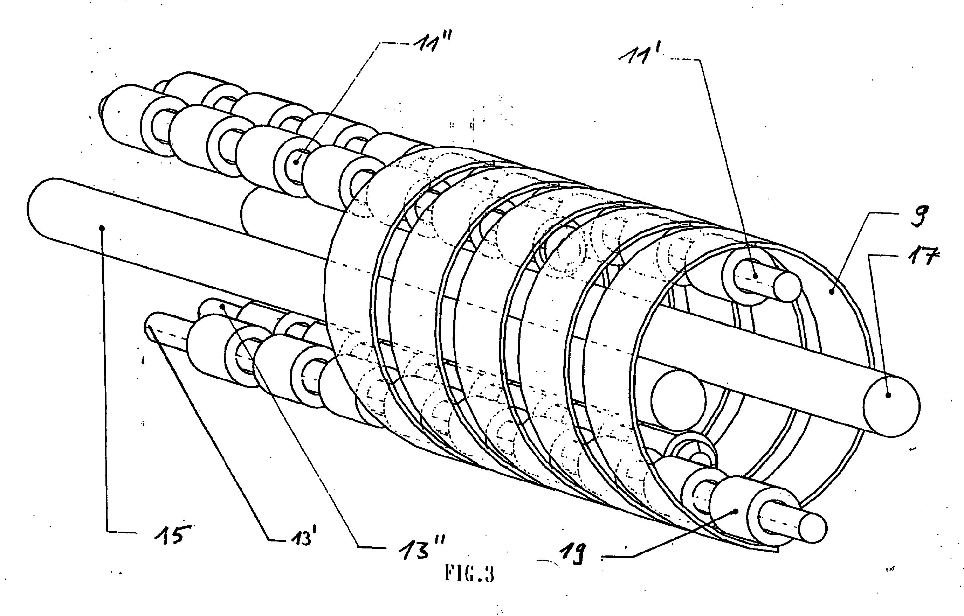

Wie aus Fig. 3 ersichtlich, besteht der Schaft 1 aus einer im Querschnitt ringförmigen Feder, insbesondere Flachfeder 9, die sich vom proximalen Ende 5 bis zum distalen Ende 3 des Schaftes 1 erstreckt. Die Feder, deren ringförmiger Querschnitt sowohl Kreisform, ovale Form als auch mehreckige Form einschließt, ist beispielsweise aus Stahl oder Kunststoff gefertigt.

Am Innenumfang bzw. der Innenwandung der Flachfeder 9 sind mehrere, beispielsweise fünf oben geführte Seile 11 bis 11'''' und fünf unten geführte Seile 13 bis 13'''' sowie seitliche Führungen links 15 und rechts 17 angeordnet.

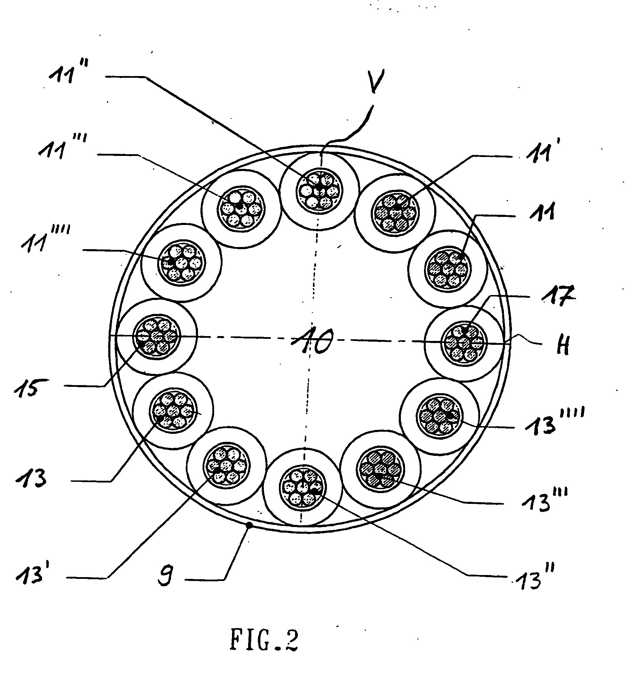

Die in Fig. 2 dargestellte Verteilung im Querschnitt der Seile 11 bis 11'''', 13 bis 13'''', 15 und 17 zeigt, dass die seitlichen Führungen im Querschnitt am Innenumfang der Flachfeder 9 einander gegenüber in einer horizontalen Mittelebene liegen bzw. auf einer horizontalen Mittelachse H angeordnet sind. Überhalb und unterhalb der Achse H sind am Innenumfang, insbesondere in äquiebene liegen bzw. auf einer horizontalen Mittelachse H angeordnet sind. Überhalb und unterhalb der Achse H sind am Innenumfang, insbesondere in äquidistalen Abständen, oben und unten geführte Seile 11 bis 11'''' und 13 bis 13'''' angeordnet.

Hierbei können, wie aus Fig. 2 ersichtlich, alle Seile als Litzen, insbesondere Stahl- oder Kunststoff gefertigt, mit beispielsweise fünf Adern, ausgebildet sein. Die oben und unten geführten Seile 11 bis 11'''' und 13 bis 13'''' verlaufen in Führungselementen 19, die ringförmig ausgebildet sein können und in Längsrichtung entlang einer Längsachse in Abständen, insbesondere in äquidistalen Abständen, entsprechend der Windung bzw. dem Gang und der Breite der Flachfeder am Innenumfang beispielsweise mittels Laserschweißen angeordnet sind.

Die Seile 11 bis 11''''und 13 bis 13'''', die in ihrem Außenumfang eine etwas geringere Abmessung als der Innenumfang der Führungselemente 19 aufweisen, sind aus dem proximalen Ende 5 des Schaftes in einen ersten kegelförmig erweiterten Bereich des in Fig. 4 dargestellten Gehäuses 6 herausgeführt und erstrecken sich bis in das im daran anschließenden erweiterten kugelförmigen Bereich angeordnete Halteteil 7. Hierbei vergrößert sich der Durchmesser der im Querschnitt ringförmigen Anordnung der geführten Seile 11 bis 11'''' und 13 bis 13'''' von wenigen Millimetern, beispielsweise 3 mm im Bereich des Schaftes (bei einem Außendurchmesser des Schaftes 1 von ca. 5 bis 6 mm) auf etwa das Doppelte. Die Seile 11 bis 11'''' und 13 bis 13'''' können in diesem Bereich, trotz ortsfest im Gehäuse 6 angeordnetem Halteteil 7, ebenfalls innerhalb Führungen, wie beispielsweise in der Zeichnung gestrichelt dargestellte starre Führungen 12 verlaufen, um nicht nur Zug-, sondern auch Schubkräfte auszuwirken.

Das Gehäuse 6 ist in seinem ersten, sich am proximalen Ende 5 des Schaftes 1 anschließenden Bereich entsprechend erweitert, beispielsweise kegelförmig ausgebildet. Im Anschluss an diesen ersten Bereich setzt sich das Gehäuses 6 in einen erweiterten Kugelbereich mit dem Halteteil 7 fort. Die geführten Seile 11 bis 11'''' und 13 bis 13'''' werden im ersten Bereich in einer starren und ortsfest im Gehäuse angeordneten Hülle geführt.

In diesem Halteteil 7 befindet sich ein in der Zeichnung nicht näher dargestellter Befestigungsmechanismus, der durch ein Betätigungselement, beispielsweise einen Bedienhebel 21, betätigt werden kann, um die proximalen Enden der Seile 11 und 13 in ihrer. Längsrichtung beweglich freizugeben bzw. zu fixieren. Eine derartige Befestigungseinrichtung kann beispielsweise als eine senkrecht zur Zeichenebene angeordnete Matrize bzw. Führungsplatte ausgebildet sein, welche Löcher zur in Längsrichtung beweglichen Aufnahme der Seile 11 bis 13 in entsprechender Geometrie, beispielsweise kreisförmig, in einer Linie oder in zwei parallelen Reihen aufweist. Selbstverständlich besitzen die Löcher in der Führungsplatte einen geringfügig größeren Innendurchmesser als der Außendurchmesser der Seile 11 bis 11'''' und 13 bis 13''''. Um die Seile in ihrer Längsrichtung zu blockieren bzw. freizugeben, kann beispielsweise eine entsprechend der ersten Führungsplatte ausgebildete an dieser anliegende zweite Führungsplatte, deren Löcher in Freigabeposition miteinander fluchten, über den Bedienhebel 21 zueinander verschoben werden. Hierbei werden die Seilenden in ihrer jeweiligen Längsposition festgeklemmt. Der Bedienhebel 21 und die zweite, als Klemmplatte fungierende Führungsplatte, welche parallel zur ersten Führungsplatte relativ zu dieser verschoben werden kann, weist somit eine Freigabe- und eine Feststellposition auf, wobei der Bedienhebel und damit die Klemmplatte in diesen Positionen beispielsweise über Rastmittel fixiert sein kann, oder dauerhaft in einer Position, insbesondere der Fixierposition, beispielsweise durch eine Feder vorgespannt sein kann, um nur für die Dauer der Betätigung des Bedienhebels in die Freigabeposition gelegt zu werden. Zumindest im Bereich ihrer Längsverschiebbarkeit innerhalb der Befestigungseinrichtung können die Seilenden als Stangen ausgebildet oder mit starren Hülsen ummantelt sein. Hierdurch wird vorteilhafterweise einer Abnützung bzw. einem Materialverschleiß, wie Spleißen der Enden, durch längere Benutzung vorgebeugt.

Wie in Fig. 4 ersichtlich ist im Bereich des an den kegelförmigen ersten Abschnitt des Gehäuses anschließenden teilkugelförmigen Abschnitts, der das Halteelement 7 beinhaltet, ein Lichtleiter vom Gehäuseinneren nach außen geführt, um an eine externe, in der Zeichnung nicht dargestellte Lichtquelle angeschlossen zu werden. Der den proximalen Abschluss des Gehäuses bildende im Durchmesser gegenüber dem kugelförmigen Abschnitt kleiner ausgebildete zylinderförmige Bereich, ist als ein nicht näher dargestelltes Okular 8 für die Bedienperson, beispielsweise der Arzt, ausgebildet. Dieses Okular 8 ist mit einem Bildleiter 25 verbunden, der sich ebenso wie der Lichtleiter 23 im Inneren des Endoskops bis zu dessen distalem Ende erstreckt.

Am distalen Ende 3 des Endoskops bzw. des Schaftes 1 befindet sich ein Endoskopkopf 27, der ebenso wie der gesamte Schaft 1 gegenüber der Umgebung abgedichtet ist.

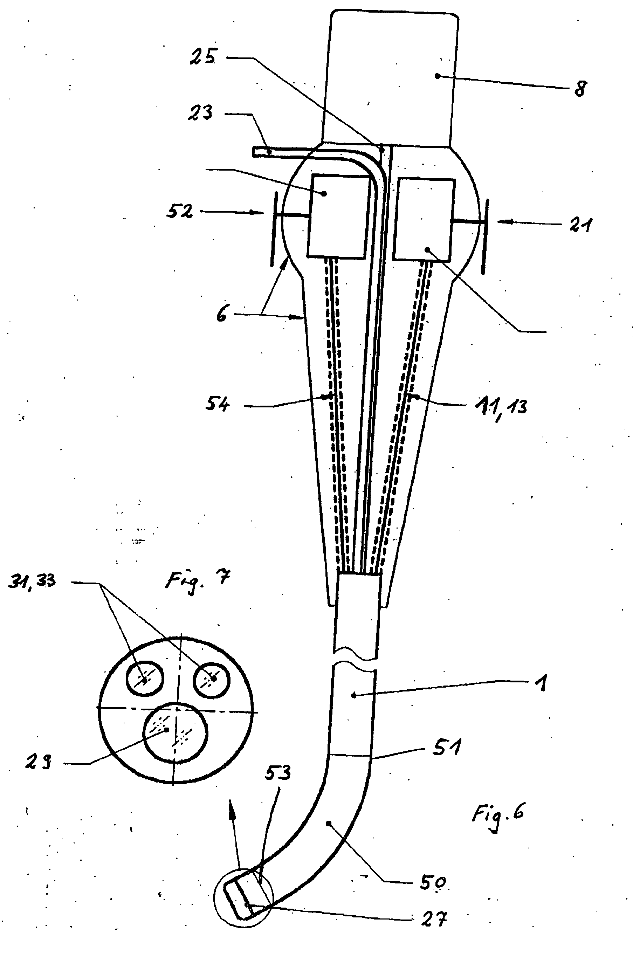

Wie aus Fig. 5 ersichtlich, beinhaltet der Endoskopkopf eine Bildlinse 29, die im Schaftinneren mit dem Bildleiter 25 verbunden ist, und zwei Lichtlinsen 31 und 33, die beide in nicht näher dargestellter Weise über eine Y-Verzweigung im Schaftinneren, welches einen Freiraum 10 (siehe Fig. 2), beispielsweise in Form eines Kanals aufweist, mit dem Lichtleiter verbunden sind.

Wie in Fig. 1 dargestellt, ist die Flachfeder 9 aus Stabilitätsgründen von einem elastischen, beispielsweise aus einem Stahlgeflecht bestehendem Netz 35 umgeben, welches von einer flexiblen Hülle 37 aus Kunststoff oder Gummi ummantelt wird. Diese Hülle 37 dichtet den Schaft 1 und eventuell auch den Kopf 27 gegenüber der Umgebung ab und sorgt vorteilhafterweise gleichzeitig für eine geringere Reibung zum Inneren eines Tubus bzw. Katheters.

Wie aus Fig. 1 ersichtlich, enden die in Führungselementen entlang einer Achse parallel zur Längsachse L geführten Seile 11 bis 11'''' und 13 bis 13'''' an ihrem dem proximalen Ende entgegengesetzten Ende in unterschiedlichen Abständen vom proximalen Ende. Hierbei sind deren Endpunkte fest mit den entsprechenden Führungselementen, beispielsweise mittels Laserschweißen, verbunden, oder weisen einen vergrößerten Kopf auf, der in distaler Richtung aus dem entsprechenden Führungselement hervorragt und in proximaler Richtung gegenüber diesem Führungseleinent als Anschlag dient.

In Fig. 4 sind aus-Gründen der Übersichtlichkeit nur die Seile 11', 11" und 13" sowie deren Enden 43, 41 und 39 dargestellt. In der in der Zeichnung dargestellten bevorzugten Ausführungsform sind die Seile, beispielsweise punktsymmetrisch oder symmetrisch zur Achse H, in Paare etwa gleicher Seillänge unterteilt, wobei die Seilpaare (beispielsweise 11; 13, 11'; 13', 11''; 13'', 11'''; 13''' und 11''''; 13'''') am Schaft 1 bzw. an der Flachfeder 9 in unterschiedlichen Abständen vom proximalen Ende 5 befestigt sind bzw. einen Anschlag in Richtung zum proximalen Ende 5, also in Zugrichtung der Seile bilden.

Die unterschiedlichen Angriffspunkte können hierbei den Schaft in äquidistale Abschnitte unterteilen, wobei im Bereich durch bestimmte Anwendungen vorhersehbarer starker Biegungen die Abstände vorteilhafterweise kleiner gewählt werden können, um die Biegung exakter zu definieren. Dagegen können die Abstände in vorhersehbar geraden Teilen bzw. Teilen mit nicht exakt gewünschter Krümmung vergrößert werden. Wie in Fig. 1 ersichtlich, stellt sich nach dem Festklemmen bzw. Fixieren der Seilenden in der Befestigungseinrichtung eine Versteifung der zuletzt im flexiblen Zustand gewählten Form ein. Hierbei sind die Seillängen der Seile 11 bis 11'''' und 13 bis 13'''' fest und somit auch die Abstände der Angriffspunkte, also der jeweiligen Seilenden 39, 41, 43 usw., vom distalen Ende. Selbstverständlich kann die Anzahl der Seile und Angriffspunkte erhöht werden, um die mögliche Dehnung bzw. Streckung zwischen in der Länge benachbarten Angriffspunkten innerhalb kleiner Toleranzen zu halten. Zudem wird die Stabilität bei der paarweisen Anordnung nicht nur durch Zug- sondern auch durch die Schubkraft des jeweiligen Partner eines Zugseils unterstützt. Weiterhin weist auch die Flachfeder 9 eine gewisse Tendenz auf, den Gesamtabstand zwischen zwei in Längsrichtung benachbarten Angriffspunkten in möglichst gleiche Abstände benachbarter Windungen bzw. in Längsrichtung benachbarter Führungselemente 19 in diesem Bereich aufzuteilen.

Da im dargestellten Ausführungsbeispiel die endoskopartige Vorrichtung zur Notfallintubation eingesetzt werden soll, ist beispielsweise aus anatomischen Gründen eine S-Form zu erreichen, in die ein vorher über den Schaft geschobener flexibler Tubus bzw. Katheter gebracht werden muß.

Diese S-Form soll innerhalb einer Ebene gebildet werden, um beim Einführen auch seitliche Kräfte ausüben zu können. Um dies zu erreichen, sind im Ausführungsbeispiel die seitlichen Führungen 15 und 17 im Gegensatz zu den geführten Seilen 11 bis 11'''' und 13 bis 13'''' nicht nur an den Endpunkten mit dem Schaft 1 bzw. der Flachfeder 9 verbunden, sondern in mehreren, vorteilhafterweise in allen, Berührungspunkten mit der Flachfeder 9 entlang ihrer Längsachse. Auf diese Weise sind die gegenüberliegenden Seiten entlang dieser Achsen in ihrer Länge weder dehn- noch komprimierbar, so dass eine Biegung des Schaftes 1 in der Ebene der beiden seitlichen Führungen 15 und 17, also in einer Ebene senkrecht zur Zeichenebene der Fig. 1, verhindert wird.

Nach dem Öffnen des Rachens mit der einen Hand mittels eines Laryngoskops wird der Schaft 1 mit dem Tubus mit der anderen Hand über die Luftröhre in die Lunge geschoben.

Ergibt sich die vorgebogene S-Form während des Einführens als nicht ideal, ist es möglich, die stabile Form durch Lösen der Befestigungsvorrichtung mittels des Bedienhebels 21 aufzulösen, so dass sich der bereits zu einem Teil eingeführte Bereich denunterschiedlichen S-Form anpasst. In dieser den anatomischen Gegebenheiten des Einzelfalls angepassten Form kann das Einführen fortgesetzt werden, bis das Erreichen des Lungeneingangs vom Arzt über das Okular 8 festgestellt wird. Anschließend wird die starre S-Form des Endoskops durch Lösen der Befestigungseinrichtung gelöst, so dass sich das nunmehr flexible Endoskop bzw. dessen Schaft 1 aus dem Tubus herausziehen lässt. Hierbei bestehen zwischen dem flexiblen Tubus und dem Schaft 1 keine oder allenfalls sehr geringe Reibungskräfte, so-dass ein ungewolltes Herausziehen des Tubus verhindert wird.

In Fig. 6 und 7 ist ein weiteres Ausführungsbeispiel einer erfindungsgemäßen endoskopartigen Vorrichtung dargestellt, welches im Unterschied zu dem vorstehend geschilderten Ausführungsbeispiel eine steuerbare Spitze 50 aufweist. Diese steuerbare Spitze 50, welche in Fig. 6 in ihrer gebogenen Stellung (nach links) dargestellt ist, erstreckt sich vom Trennstrich 51 bis zum Trennstrich 53 so dass der Endoskopschaft 1 nicht wie im vorstehend geschilderten Ausführungsbeispiel direkt in den Endoskopkopf 27 übergeht, sondern sich zwischen Ende des Schafts 1 (Trennlinie 51) und Beginn des Endoskopkopfs 27 (Trennlinie 53) die steuerbare Endoskopspitze 50 befindet. Diese ist in ihren Längenabmessungen im Verhältnis zur Länge des Endoskopschafts 1 wesentlich kürzer, beispielsweise in einem Verhältnis von ungefähr 1:10 mit einer Länge der Spitze von 2 bis 4 cm, insbesondere 3 cm bei einer Schaftlänge von beispielsweise 30 bis 40 cm.

Die Schaftlänge entspricht hierbei der Länge eines üblichen Tubus, beispielsweise Luftröhrenkatheters, welcher bei der Notfallintubation eingesetzt werden soll. Die Spitze 50 kann vorteilhafterweise über den über den Schaft 1 geschobenen Katheter hinausragen, da der Katheter in einem solch kurzen Bereich (2 bis 4 cm) relativ starr ist und eine Bewegung der Endoskopspitze 50 stark behindern oder gar verhindern würde. Die Bewegung der Endoskopspitze 50 über beispielsweise als Bowdenzugseile 54 ausgebildete Steuermittel, welche in der Zeichnung nur schematisch dargestellt sind, erfolgt über eine im proximalen Bereich angeordnete, beispielsweise als Steuerhebel 52 ausgebildete, Steuereinrichtung, wobei diese Steuerung bzw. die Beweglichkeit der Endoskopspitze 50 durch herkömmliche Mechanismen, wie sie zur Steuerung eines Endoskopschafts flexibler Endoskope verwendet werden, erfolgen kann.

Im übrigen ist diese weitere Ausführungsform abgesehen von der steuerbaren Spitze sowie die Mittel zu deren Steuerung 52, 54 ebenso wie das vorstehend geschilderte Ausführungsbeispiel ausgebildet, so dass diese Ausführungsform alle Vorteile der vorstehenden Ausführungsform aufweist und zusätzlich im kleinen Spitzenbereich durch die Ausbildung einer steuerbaren Spitze 50 steuerbar ist. Hierdurch können vorteilhafterweise in Extremfällen, wie stark verwinkelte Luftröhre oder eine gerissene Luftröhre bei einem Einführen eines über den Schaft 1 geschobenen Katheters, von einer Bedienperson, in der Regel dem Notarzt, an der Spitze Steuerbewegungen ausgeführt werden, so dass sich das Einführen erleichtert.

Beispielsweise ist es hierdurch möglich, bei einer gerissenen Luftröhre die an der Reißstelle einen Versatz von mehreren Millimetern, beispielsweise 5 bis 10 mm aufweist, bei einem Einführen bzw. einer Intubation diesen Versatz durch eine entsprechende Bewegung der Endoskopspitze 50 über den Hebel 52 auszugleichen und die Spitze und somit nachfolgend den über den Schaft 1 gestülpten Katheter in den so versetzten Teil der Luftröhre einfach und schnell einzuführen.

Durch diese flexible steuerbare Spitze 50 wird aber auch für weniger erfahrene Ärzte das Einführen eines Luftkatheters, insbesondere bei einem Notfallpatienten, wesentlich erleichtert, da während des Einführens jederzeit über die Optik der weitere Weg innerhalb der evtl. verletzten Luftröhre beobachtet und entsprechend die Spitze 50 an unerwartete oder extreme Situationen angepasst werden kann. Durch die geringe Abmessung der flexiblen steuerbaren Spitze 50, welche selbstverständlich wie übliche herkömmliche flexible Endoskopschafte nicht festgestellt und somit nicht wie der Schaft 1 in einen (gegen beim Einführen auftretende Kräftezund Drücke) starren Zustand versetzt werden kann, bleiben die vorstehend für das-erste Ausführungsbeispiel geschilderten Vorteile eines feststellbaren starren Schafts 1 erhalten, nämlich das manuelle (durch Hände einer Bedienperson, insbesondere des Notarztes) Vorbiegen des Schaftes 1 in eine entsprechende Form, die Fixierung dieser Form, so dass beim Einführen keine Stauchung (und eventuelle Beschädigung der Vorrichtung, beispielsweise der Optik) des Schaftes 1 erfolgen kann und Lösen dieses starren Zustandes um das Endoskop aus dem eingesetzten Katheter möglichst leicht und reibungsfrei herauszuziehen oder auch Lösen während des Einführens (hierdurch Anpassung an die vorhandene Windung der Luftröhre) und erneute Fixierung dieses angepassten Zustandes des Schaftes 1 um weiter Druck bzw. Kraft im wesentlichen in Richtung der Längsrichtung, also Einführrichtung, des Endoskops mit Katheters ohne die Gefahr einer Stauchung des Schaftes 1 zu ermöglichen.

In der bevorzugten Ausführungsform ist die Endoskopspitze 50 nur in einer Ebene, beispielsweise wie dargestellt in der Zeichenebene, auf- und ab- bzw. nach links und nach rechts schwenkbar, wobei sich diese Bewegung vorteilhafterweise bereits durch eine geringe Anzahl von Steuerseilen 54 erreichen läßt und der Querschnitt des Schaftes 1 und der Spitze 50 entsprechend klein gehalten werden kann. Durch Drehung der gesamten Vorrichtung um ihre Längsachse können jedoch beliebige Richtungen erreicht werden, wobei eine solche Drehung selbst im Einsatz der Notfallintubation in einem gewissen Bereich ohne Verletzung der Luftröhre möglich ist. Selbstverständlich ist es auch denkbar bei entsprechenden Abmessungen weitere Steuerseile 54 im Endoskopschaft 1 bis zur Spitze 50 unterzubringen, so dass eine Steuerbarkeit der Endoskopspitze 50 in allen Richtungen, wie bei herkömmlichen flexiblen Endoskopen, ermöglicht wird. Da in der bevorzugten Ausgestaltung der Erfindung die Formbarkeit des Schaftes 1 ebenfalls auf eine Bewegung (beispielsweise S-förmig) innerhalb derselben Ebene wie die Steuerbarkeit der Spitze 50 eingeschränkt sein kann, ist die Starrheit in anderen Ebenen und damit die Unempfindlichkeit gegen Stauchungen oder Verwindungen, insbesondere bei einer Drehung um die Längsachse der Vorrichtung besonders hoch.

Die Erfindung ist jedoch nicht auf die Endoskopie, insbesondere Notfallintubation, begrenzt, sondern für alle endoskopartigen Vorrichtungen anwendbar. Es wird ausdrücklich darauf hingewiesen, dass dieser Begriff nach der Erfindung weitreichend zu verstehen ist, wobei beispielsweise auch biegsame Wellen in der Werkzeugtechnik, insbesondere für Verlängerungen von Schraubendrehern, Bohrern u.ä., und allgemeine biegsame Glieder, die zumindest in Teilbereichen flexibel bzw. biegsam ausgebildet sind und bei denen eine Versteifung auf eine beliebige vorbestimmte Form erwünscht ist, unter diese Definition zu subsumieren sind. Vorteilhafterweise kann die vorstehend beschriebene Formbarkeit und Flexibilität hierbei über den gesamten Schaft 1 vorhanden sein. Die Starrheit in der festgestellten Position ermöglicht ein vorher formbares Gebilde, dass nachfolgend dieses Gebilde gegen beispielsweise beim Einführen in eine Luftröhre auftretende Drücke und Kräfte unempfindlich ist und eventuelle hieraus folgende Stauchungen vermieden werden können. Im Unterschied zu flexiblen Endoskopen mit steuerbarem Schaft, beispielsweise Bronchoskop, bleibt die starre einmal eingenommen Form aufrecht erhalten (bis zum Lösen der Befestigungseinrichtung), so dass Beschädigungen durch eine Stauchung, wie beispielsweise Beschädigung der Optik und kostspielige Reparaturen vom mehreren Tausend DM, vermieden werden können.

## Patentansprüche

1. Endoskop für die Notfallintubation, mit einem Halteteil (7) und einem wenigstens in Teilbereichen flexibel ausgebildeten Schaft (1), wobei das Endoskop eine Endoskopspitze (50) aufweist, deren Bewegung über Steuermittel (54) und einer im proximalen Bereich angeordneten Steuereinrichtung (52) steuerbar ist,
**dadurch gekennzeichnet, dass**
wenigstens zwei längliche biegbare Zug- und/oder Schubmittel am Schaft (1) in axialer Richtung in unterschiedlichen Abständen vom proximalen Ende angreifen, wobei sich die Zug- und/oder Schubmittel bis an das proximale Ende erstrecken und dort in einer sich im Halteteil (2) befindenden Befestigungseinrichtung mit einer Freigabe- und Feststellposition feststellbar und lösbar gelagert sind,
wobei die proximalen Enden der Zug- und/oder Schubmittel in der Freigabeposition in ihrer Längsrichtung frei beweglich sind, so dass der Schaft (1) von Hand formbar ist und in der Feststellposition in ihrer jeweiligen Längsposition festgeklemmt sind.

2. Vorrichtung nach Anspruch 1, **dadurch gekennzeichnet, dass** der Schaft (1) einen ringförmigen Querschnitt aufweist und als Flachfeder (9) ausgebildet ist.

3. Vorrichtung nach Anspruch 1 oder 2, **dadurch gekennzeichnet, dass** die Zug- und/oder Schubmittel als in Längsrichtung im Wesentlichen starre Seile ausgebildet sind.

4. Vorrichtung nach einem der vorhergehenden Ansprüche, **dadurch gekennzeichnet, dass** die Zug- und/oder Schubmittel innerhalb des Schaftes (1) in Führungselementen (19) in Längsrichtung beweglich gelagert sind.

5. Vorrichtung nach einem der vorhergehenden Ansprüche, **dadurch gekennzeichnet, dass** die Zug- und/oder Schubmittel innerhalb des Schaftes (1) im Querschnitt ringförmig an dessen Innenumfang gelagert sind.

6. Vorrichtung nach einem der vorhergehenden Ansprüche, **dadurch gekennzeichnet, dass** die Zug- und/oder Schubmittel am Schaft (1) in Zug- und Schubrichtung begrenzt angreifen.

7. Vorrichtung nach einem der vorhergehenden Ansprüche, **dadurch gekennzeichnet, dass** innerhalb des Schaftes (1) im Querschnitt gegenüberliegende, mit dem Schaft (1) wenigstens in Teilbereichen festverbundene seitliche Führungen angeordnet sind.

8. Vorrichtung nach einem der vorhergehenden Ansprüche, **dadurch gekennzeichnet, dass** der Schaft (1) im Inneren einen Kanal (10) umfasst.

9. Vorrichtung nach Anspruch 8, **dadurch gekennzeichnet, dass** der Kanal (10) als Kanal für einen Lichtleiter (23) und einen Bildleiter (25) und/öder als Instrumentenkanal ausgebildet ist.

10. Vorrichtung nach einem der vorhergehenden Ansprüche, **dadurch gekennzeichnet, dass** Zug- und/oder Schubmittel am Schaft (1) in axialer Richtung paarweise in im Wesentlichen gleichen Abständen vom proximalen Ende angreifen.

11. Vorrichtung nach einem der vorhergehenden Ansprüche, **dadurch gekennzeichnet, dass** die Enden der Zug- und/oder Schubmittelpaare am Schaft (1) punktsymmetrisch zum Schaftmittelpunkt oder symmetrisch zur horizontalen H oder vertikalen Achse V angeordnet sind.

## Revendications

1. Endoscope pour intubation d'urgence, comportant une partie de retenue (7) et une tige (1) conformée de manière souple au moins dans des zones partielles, l'endoscope présentant une pointe d'endoscope (50) dont le mouvement peut être commandé via un moyen de commande (54) et un dispositif de commande (52) agencé dans la zone proximale,
**caractérisé en ce que**
au moins deux moyens de traction et/ou de poussée longitudinaux flexibles s'appliquent sur la tige (1) dans la direction axiale à différentes distances de l'extrémité proximale, les moyens de traction et/ou de poussée s'étendant jusqu'à l'extrémité proximale et y étant montés dans un dispositif de fixation se trouvant dans la partie de retenue (2) avec une position de libération et une position de blocage de manière à pouvoir être bloqués et dégagés,
dans lequel les extrémités proximales des moyens de traction et/ou de poussée peuvent être déplacées librement dans la position de libération dans sa direction longitudinale de sorte que la tige (1) soit déformable à la main, et sont bloquées solidement en position de blocage dans leur position longitudinale respective.

2. Dispositif selon la revendication 1, **caractérisé en ce que** la tige (1) présente une section transversale annulaire et se présente sous la forme d'un ressort à lame (9).

3. Dispositif selon la revendication 1 ou 2, **caractérisé en ce que** les moyens de traction et/ou de poussée se présentent sous la forme de câbles sensiblement rigides dans la direction longitudinale.

4. Dispositif selon l'une quelconque des revendications précédentes, **caractérisé en ce que** les moyens de traction et/ou de poussée à l'intérieur de la tige (1) sont montés dans des éléments de guidage (19) de manière à pouvoir se déplacer dans la direction longitudinale.

5. Dispositif selon l'une quelconque des revendications précédentes, **caractérisé en ce que** les moyens de traction et/ou de poussée à l'intérieur de la tige (1) sont montés en forme d'anneau en section transversale sur sa circonférence interne.

6. Dispositif selon l'une quelconque des revendications précédentes, **caractérisé en ce que** les moyens de traction et/ou de poussée sont appliqués sur la tige (1) de manière limitée dans le sens de la traction et de la poussée.

7. Dispositif selon l'une quelconque des revendications précédentes, **caractérisé en ce qu'**il est agencé, à l'intérieur de la tige (1), des glissières latérales opposées dans la section transversale, raccordées de manière fixe à la tige (1), au moins dans des zones partielles.

8. Dispositif selon l'une quelconque des revendications précédentes, **caractérisé en ce que** la tige (1) comprend un canal (10) à l'intérieur.

9. Dispositif selon la revendication 8, **caractérisé en ce que** le canal se présente sous la forme d'un canal (10) pour un conducteur optique (23) et un conducteur d'image (25) et/ou sous la forme d'un canal instrumental.

10. Dispositif selon l'une quelconque des revendications précédentes, **caractérisé en ce que** les moyens de traction et/ou de poussée sont appliqués sur la tige (1) dans la direction axiale par paires à des distances sensiblement identiques de l'extrémité proximale.

11. Dispositif selon l'une quelconque des revendications précédentes, **caractérisé en ce que** les extrémités des paires de moyens de traction et/ou de poussée sont agencées sur la tige (1) en symétrie ponctuelle par rapport au centre de la tige ou symétriquement à l'axe horizontal H ou à l'axe vertical V.

## Claims

1. Endoscope for emergency intubation, with a holding portion (7) and a shaft (1) which is configured to be flexible at least in partial areas, the endoscope comprising an endoscope tip (50) the movement of which being controllable via control means (54) and a control device (52) arranged in the proximal region,
**characterised in that**
at least two longitudinally bendable pulling and/or pushing means act on the shaft (1) in the axial direction at different distances from the proximal end, the pulling and/or pushing means extending as far as the proximal end and being lockably and releasably mounted there in a fixing device located in the holding portion (2) with a released and locked position,
the proximal ends of the pulling and/or pushing means being freely movable in their longitudinal direction in the released position, so that the shaft (1) may be shaped manually, and are clamped in the locked position in their respective longitudinal position.

2. Device according to claim 1, **characterised in that** the shaft (1) has a ring-shaped cross-section and is configured as a leaf spring (9).

3. Device according to claim 1 or 2, **characterised in that** the pulling and/or pushing means are configured as substantially rigid cables in the longitudinal direction.

4. Device according to any one of the preceding claims, **characterised in that** the pulling and/or pushing means within the shaft (1) are movably mounted in guide elements (19) in the longitudinal direction.

5. Device according to any one of the preceding claims, **characterised in that** the pulling and/or pushing means are mounted in the shaft (1) with an annular cross-section on its inner circumference.

6. Device according to any one of the preceding claims, **characterised in that** the pulling and/or pushing means act on the shaft (1) to a limited extent in the pulling and pushing direction.

7. Device according to any one of the preceding claims, **characterised in that** lateral guides opposing one another in cross-section are arranged within the shaft (1) and are fixedly attached to the shaft (1) at least in partial areas.

8. Device according to any one of the preceding claims, **characterised in that** the shaft (1) comprises a channel (10) in the interior.

9. Device according to claim 8, **characterised in that** the channel (10) is configured as a channel for a light guide (23) and an image guide (25) and/or as a channel for instruments.

10. Device according to any one of the preceding claims, **characterised in that** the pulling and/or pushing means act in pairs on the shaft (1) in the axial direction at substantially equal distances from the proximal end.

11. Device according to any one of the preceding claims, **characterised in that** the ends of the pulling and/or pushing means are arranged on the shaft (1) point symmetrically to the midpoint of the shaft or symmetrically to the horizontal axis H or vertical axis V.
